# EUROPÄISCHE PATENTSCHRIFT

(11) **EP 2 060 556 B1**
(45) Veröffentlichungstag und Bekanntmachung des Hinweises auf die Patenterteilung: **11.11.2009**
(21) Anmeldenummer: 07120946.4
(22) Anmeldetag: 19.11.2007
(51) Int. Cl.: C07C 51/34, C07C 65/21, A61K 8/06, A01N 37/40

(54) **Verfahren zur Herstellung von 4-Methoxybenzoesäure aus pflanzlichem Anethol und dessen Verwendung in kosmetischen oder dermatologischen Produkten sowie Lebensmitteln.**
Method for manufacturing 4-methoxybenzoic acid from vegetable anethol and its application in cosmetic or dermatological products and foodstuffs
Procédé de fabrication de 4-méthoxybenzoïques à partir d'anéthol végétal et son utilisation dans les produits cosmétiques ou dermatologiques ainsi que des aliments

(43) Veröffentlichungstag der Anmeldung: 20.05.2009
(73) Patentinhaber: Dr. Straetmans Chemische Produkte GmbH, 22143 Hamburg (DE)
(72) Erfinder: Jänichen, Jan Dr., 22143 Hamburg (DE); Petersen, Wilfried Dr., 22143 Hamburg (DE); Jenny, Rudolf, 3112 Allmendingen (CH); Nobis, Markus Dr., 3250 Lyss (CH)
(74) Vertreter: Raffay & Fleck

(56) Entgegenhaltungen:
- EP-A- 1 325 731
- US-A- 3 799 940
- BRINER, E. ET AL: "Researches on the infrared absorption spectra of ozonides . XV. Ozonation of trans- and cis-anethole; autoxidation, accelerated by ozone , of anisic aldehyde" HELVETICA CHIMICA ACTA , 41, 2178-85 CODEN: HCACAV; ISSN: 0018-019X, 1958, XP002476887
- DATABASE CA [Online] CHEMICAL ABSTRACTS SERVICE, COLUMBUS, OHIO, US; LIU, LIHUA ET AL: "Preparation of p-anisic aldehyde by ozonization" XP002476890 gefunden im STN Database accession no. 1998:290975 & GUANGXI HUAGONG , 26(1), 11-14 CODEN: GUHUF2; ISSN: 1003-0840, 1997,
- CAREY AND SUNDBERG: "Advanced Organic Chemistry, second edition, Part B: Reactions and Synthesis" 1985, PLENUM PUBLISHING CORPORATION , NEW YORK , XP002476889 * Seite 513, Zeilen 5-7 *
- CAREY; SUNDBERG: 'Advanced Organic Chemistry, second edition, Part B: Reactions and Synthesis', 1985, PLENUM PUBLISHING CORPORATION, NEW YORK * Seiten 510-514 *
- HENNE A.L.; HILL P.: 'The Preparation of Aldehydes, Ketones, and Acids by Ozone Oxidation' JOURNAL OF THE AMERICAN CHEMICAL SOCIETY Bd. 65, 1943, Seiten 752 - 754

## Beschreibung

### Technisches Gebiet

Die Erfindung betrifft ein Verfahren zur Herstellung von 4-Methoxybenzoesäure.

### Stand der Technik

Helvetica Chimica Acta 41, 2178-85, 1958 offenbart die Umsetzung von trans- und cis-Anethol mit Ozon zu 4-Methoxybenzaldehyd und die weitere Oxidation zu 4-Methoxybenzoesäure.

Carey und Sundberg "Advanced Organic Chemistry, 2nd ed., Part B: Reaction and Synthesis" 1985, Plenum publ. Corp., New York S. 510-513 offenbart die oxidative, *in situ,* Aufarbeitung zur Herstellung von Carbonsäuren in einer sauren Ozonolysereaktion.

Die Suche nach Alternativen zur Ausbeutung fossiler Ressourcen zur Energiegewinnung oder zur Herstellung chemischer Grundstoffe hat in den vergangenen Jahren stetig an Bedeutung gewonnen. Das Pflanzenreich nimmt bei der Suche nach alternativen Rohstoffquellen eine herausragende Position ein, da es komplexe Grundstoffe in nachwachsender Form liefert, die sich bei ihrem Abbau obendrein auch noch klimaneutral verhalten. Auch vor dem Hintergrund einer wachsenden Rückbesinnung vieler Menschen auf die Natur und der Endlichkeit fossiler Quellen hat sich daher ein immer größer werdender Markt für Produkte auf Basis natürlicher Rohstoffe und damit verbunden eine wachsende Industrie zur deren Gewinnung und Verarbeitung entwickelt.

Bei einer ökologischen Beurteilung der Rohstoffe spielt, neben deren Herkunft, auch deren mögliche Prozessierung eine wichtige Rolle. So sind im Rahmen einer gesamtheitlichen Betrachtung, auch der Einsatz von Energieressourcen und die Produktion von Abfallprodukten bei deren Weiterverarbeitung zu berücksichtigen, und in eine ökologische Bilanz einzubeziehen. In verschiedenen Industrien wird mittlerweile die natürliche Herkunft der verwendeten Rohstoffe gezielt ausgelobt, und es werden verbindliche Standards zur Gewinnung und Weiterverarbeitung natürlicher Substrate definiert. So hat sich z.B. in der kosmetischen aber auch in der Nahrungsmittelindustrie eine schnell wachsende Anzahl von Anbietern natürlicher Produkte gebildet, welche die einsetzbaren Rohstoffe und Prozesse zu deren Gewinnung und Herstellung nach strengen Standards regelt.

p-Methoxybenzoesäure hat in den vergangenen Jahren als multifunktioneller Rohstoff in der kosmetischen Industrie (INCI: p-anisic acid) und in der Lebensmittelindustrie eine wachsende Bedeutung erlangt. Neben seiner Hauptfunktion als Maskierungsagens und Aromakomponente haben die interessanten Eigenschaften dieses Rohstoffes zur biologischen Stabilisierung von kosmetischen und dermatologischen Formulierungen und als Wirkstoff gegen spezifische Keime in Produkten der Haut- und Haarpflege ein großes Interesse an diesem Rohstoff geweckt. In der Natur kann p-Methoxybenzoesäure in verschiedenen Pflanzen wie z.B. Anis oder Fenchel nachgewiesen werden. Die geringen Mengen machen jedoch eine Isolierung dieses Rohstoffs aus diesen Quellen uninteressant und das derzeit von der kosmetischen oder der Nahrungsmittelindustrie eingesetzte Produkt wird aus petrochemischen Rohstoffen gewonnen.

### Darstellung der Erfindung

Vor diesem Hintergrund ist es die Aufgabe der vorliegenden Erfindung, ein ökonomisches Verfahren zur Herstellung von p-Methoxybenzoesäure mit hoher Ausbeute, welches den ökologischen Anforderungen der Hersteller natürlicher Produkte insbesondere Kosmetikprodukte entsprechen.

Überraschenderweise wurde gefunden, dass sich diese Aufgabe durch die Umsetzung natürlichen Anethols mit Ozon und nachfolgender in-situ oxidativer Aufarbeitung mit basischem Wasserstoffperoxid in vorzüglicher Weise lösen lässt.

Anethol ist ein weit verbreiteter Naturstoff, der aus verschiedenen Pflanzen durch Extraktion gewonnen werden kann. Ohne die vorliegende Patentanmeldung auf eine bestimmte Rohstoffquelle einzuschränken, dienen als Hauptquelle des Anethols, welches erfindungsgemäß verwendet werden können, die Öle des Sternanis (*Illicium Verum*) oder des Indischen Basilikums (*Ocimum Tenuiflorum*)*.* Sein charakteristischer Anisgeruch und seine geringe Toxizität machen natürliches Anethol zu einem Aromastoff, der in großen Mengen in der Lebensmittelindustrie verarbeitet wird.

Chemisch gesehen besteht natürliches Anethol aus einem cis/trans-Gemisch des 1-Methoxy-4-(1-propenyl)benzols, wobei das trans-Isomer mit etwa 98% Anteil deutlich dominiert. Während die Methoxyfunktion dieses Moleküls chemisch gesehen relativ inert ist, ermöglicht die olefinische Doppelbindung eine Reihe chemischer Modifikationen.

Die Umsetzung organischer Substrate mit Ozon stellt ein besonders interessantes, weil umweltschonendes Verfahren zur Modifikation chemischer Strukturen dar. Die reaktive Spezies Ozon kann elektrochemisch aus Sauerstoff hergestellt werden und die Bildung problematischer halogenorganischer Reaktions- oder Nebenprodukte ist ausgeschlossen, wenn die eingesetzten Substrate halogenfrei sind.

Auch werden die Vorteile des Ozons zunehmend in der Umwelttechnik eingesetzt, wo es z.B. bei der Abwasseraufbereitung oder zur chlorfreien Sterilisierung von Wasser zunehmend Einsatz findet.

In den vergangenen Jahren ist daher die Anzahl von Patentanmeldungen, welche die Verwendung von Ozon beschreiben, kontinuierlich gestiegen.

So beschreibt die Anmeldeschrift EP1362840 die Herstellung aromatischer Säuren durch katalytische Oxidation von Methylaromaten mit Ozon und Übergangsmetallkatalysatoren. In dieser Reaktion fungiert das Ozon als Oxidationsmittel welches gemeinsam mit dem Übergangsmetallkatalysator die Methylgruppe in eine Carboxylgruppe überführt. EP 1569885 beschreibt die in-situ Umsetzung von Ozoniden aus Alkenen zu Aldehyden und Ketonen ebenfalls mit Hilfe von Übergangsmetallkatalysatoren. Eine elegante Möglichkeit, Ketone zu erzeugen besteht in der Ozonolyse von Allylalkoholen und wird in der Anmeldeschrift EP1710224 beschrieben.

Die Umsetzung von Alkenen mit Ozon wird in der einschlägigen organisch chemischen Literatur ausführlich beschrieben. Die Reaktion führt dabei in einer 2+3-Cycloaddition zu einem Ozonid, welches je nach Aufarbeitung in verschiedene Funktionalitäten überführt werden kann. Die hohe Aktivität des Ozons führt jedoch in vielen Fällen zu Nebenprodukten, die nur durch genau definierte Reaktionsbedingungen kontrolliert werden können. Darüber hinaus wird mit dem Ozonid ein sehr energiereiches Intermediat gebildet, welches hohe Sicherheitsanforderungen an die gewählte Prozessführung stellt.

Unter Berücksichtigung dieser Vorbedingungen wurde nun ein Verfahren gefunden, mit dessen Hilfe ein Ozonid, welches aus einem natürliches Gemisch von cis- und trans-Anethol in einer überraschend sauberen Reaktion gebildet wurde, in-situ und in einem kontrollierbaren Prozess ohne Zusatz weiterer Katalysatoren in 4-Methoxy-benzoesäure überführt werden kann.

Die Addition des Ozons an das natürliche Anethol erfolgt dabei vorteilhafterweise bei Temperaturen zwischen -30°C und +30°C in einem Lösungsmittel aus der Gruppe der C1-6 Mono- der Dicarbonsäuren, Ester, Wasser, Aceton oder C1-6 Alkohole oder in Gemischen aus diesen genannten Lösungsmitteln. Besonders vorteilhaft erfolgt die Umsetzung in Ethanol.

Die erfindungsgemäße in-situ Umsetzung des gebildeten Ozonids zur 4-Methoxy-benzoesäure erfolgt durch oxidative Spaltung des Ozonids mit alkalischer Wasserstoffperoxidlösung. Als besonders vorteilhaft und sicher hat sich dabei erwiesen, eine Mischung des Ozonids des 1-Methoxy-4-(1-propenyl)benzol und einer konzentrierten Wasserstoffperoxidlösung zu einer erwärmten, verdünnten Lösung eines Alkali- oder Erdalkalihydroxidshinzuzusetzen. Als besonders geeignet haben sich dabei die Hydroxide des Natriums oder des Kaliums erwiesen. Eine wässrig-alkoholische Mischung des Ozonids des 1-Methoxy-4-(1-propenyl)benzols und des Wasserstoffperoxids erwies sich überraschenderweise über einen breiten Temperaturbereich als thermisch stabil. Die exotherme Spaltung des Ozonids des 1-Methoxy-4-(1-propenyl)benzols und die nachfolgende Oxidation erfolgen auf diese erfindungsgemäße Art und Weise schnell und ohne Aufkonzentrierung reaktiver Zwischenprodukte, so dass eine gute Reaktionskontrolle und hohe Prozesssicherheit erzielt werden. Als besonders vorteilhaft hat sich dabei erwiesen, das Gemisch aus Ozonid und Wasserstoffperoxid zu einer 30 - 105°C warmen 1-25 %igen Natronlaugelösung bei konstanter Temperatur hinzuzufügen.

Aus der alkalischen Reaktionsmischung kann die 4-Methoxybenzoesäure mit Hilfe von Mineralsäuren in guter Reinheit ausgefällt werden. Erfindungsgemäße Mineralsäuren werden dabei vorzugsweise aus der Gruppe Schwefelsäure oder Phosphorsäure gewählt. Besonders vorteilhaft ist es, die 4-Methoxybenzoesäure durch den Zusatz von Schwefelsäure bei einem pH-Wert zwischen 3 und 6 zu fällen. Spuren von Aldehyden oder anderen Nebenprodukten können, wenn gewünscht, durch eine Behandlung mit Aktivkohle eliminiert werden. Die aus dem beschriebenen Prozess gewonnen Ausbeute von 4-Methoxybenzoesäue beträgt zwischen 70 und 80% der Theorie.

Das erfindungsgemäße Endprodukt zeichnet sich durch eine hohe Reinheit aus, welche den Anforderungen an Rohstoffen für die kosmetische und der Nahrungsmittelindustrie voll genügt. 4-Methoxybenzoesäure eignet sich in hervorragender Weise zur Beduftung, Aromatisierung, pH-Regulierung, Entzündungshemmung und/oder Bekämpfung von Mikroorganismen in kosmetischen oder dermatologischen Produkten sowie als Wirkstoff gegen spezifische pathogene Keime in Produkten zur Behandlung der Haut und/oder zur Aromatisierung und/oder Konservierung von Nahrungsmittel oder anderen verderblichen Produkten. Vergleichende Untersuchungen mit 4-Methoxybenzoesäure petrochemischen Ursprungs zeigten keinerlei Abweichungen in den ausgelobten Eigenschaften des Produktes.

## Patentansprüche

1. Verfahren zur Herstellung von 4-Methoxybenzoesäure aus 1-Methoxy-4-(1-propenyl)benzol (Anethol) **dadurch gekennzeichnet, dass** das Ozonid des Anethols *in situ* durch eine oxidative Aufarbeitung mit basischem Wasserstoffperoxid in die 4-Methoxy-benzoesäure überführt wird.

2. Verfahren gemäß Anspruch 1 **dadurch gekennzeichnet, dass** der Ausgangsstoff 1-Methoxy-4-(1-propenyl)benzol (Anethol) durch extraktive oder destillative Prozesse aus pflanzlichen Quellen gewonnen wird.

3. Verfahren gemäß Anspruch 1 **dadurch gekennzeichnet, dass** die Bildung des Ozonids aus dem 1-Methoxy-4-(1-propenyl)benzol (Anethol) bei Temperaturen zwischen -30°C und + 30°C in einem Lösungsmittel aus der Gruppe der C1-6 Mono- der Dicarbonsäuren, Ester, Wasser, Aceton oder C1-6 Alkohole oder in Gemischen aus diesen genannten Lösungsmitteln erfolgt.

4. Verfahren gemäß Anspruch 1 **dadurch gekennzeichnet, dass** die Bildung des Ozonids aus dem 1-Methoxy-4-(1-propenyl)benzol (Anethol) bei Temperaturen zwischen -10°C und + 10°C in Ethanol erfolgt.

5. Verfahren gemäß Anspruch 1 **dadurch gekennzeichnet, dass** zur oxidativen Spaltung des Ozonids des 1-Methoxy-4-(1-propenyl)benzols dieses mit Wasserstoffperoxid-Lösung gemischt und anschließend zu einer 30 - 105°C warmen, verdünnten Lösung eines Alkali- oder Erdalkalihydroxids zugesetzt wird.

6. Verfahren gemäß Anspruch 1 **dadurch gekennzeichnet, dass** zur oxidativen Spaltung des Ozonids des 1-Methoxy-4-(1-propenyl)benzols dieses mit Wasserstoffperoxid-Lösung gemischt und anschließend zu einer 60 - 80°C warmen, 1 - 25 %igen Natronlauge zugesetzt wird.

7. Verfahren gemäß Anspruch 1 **dadurch gekennzeichnet, dass** die 4-Methoxybenzoesäure aus dem Reaktionsgemisch der oxidativen Ozonidspaltung durch Fällung mit einer Mineralsäure in einem pH-Bereich von 3 - 6 ausgefällt und durch Filtration abgetrennt wird.

8. Verfahren gemäß Anspruch 1 **dadurch gekennzeichnet, dass** das Reaktionsgemisch zur Entfernung von Nebenprodukten nach der oxidativen Spaltung des Ozonids und vor der Fällung durch eine Mineralsäure mit Aktivkohle behandelt wird.

9. Verfahren gemäß Anspruch 1 **dadurch gekennzeichnet, dass** die aus der Fällung mit einer Mineralsäure gewonnene 4-Methoxybenzoesäure durch Lösen in verdünnter Natronlauge, Behandlung mit Aktivkohle und erneute Fällung mit Schwefelsäure weiter aufgereinigt wird.

## Claims

1. A method for manufacturing 4-methoxybenzoic acid from 1 -methoxy-4-(l -propenyl) benzol (anethole) **characterised in that** the anethole ozonide is converted *in situ* by oxidising conversion with basic hydrogen peroxide in 4-methoxybenzoic acid.

2. A method according to claim 1 **characterised in that** the starting material 1-methoxy-4-(I-propenyl)benzol (anethole) is obtained using extraction or distillation processes from vegetal sources.

3. A method according to claim 1 **characterised in that** the formation of ozonide from 1-methoxy-4-(1-propenyl)benzol (anethole) takes place at temperatures between -30°C and +30°C in a solvent from the group of the C1-6 mono or dicarbon acids, ester, water, acetone or Cl-6 alcohols or in mixtures from these solvents aforementioned.

4. A method according to claim 1 **characterised in that** the formation of ozonide from 1-methoxy-4-(1-propenyl)benzol (anethole) takes place at temperatures between -10°C and +10°C in ethanol.

5. A method according to claim 1 **characterised in** for providing an oxidising separation of ozonide from 1-methoxy-4-(I-propenyl)benzol said ozonide is mixed with a hydrogen peroxide solution and then added to a 30-105°C warm, diluted solution of an alkaline or alkaline earth hydroxide.

6. A method according to claim 1 **characterised in** for providing an oxidising separation of ozonide from 1-Methoxy-4-(1-propenyl)benzol said ozonide is mixed with a hydrogen peroxide solution and then added to a 60-80°C warm, 1-25% caustic soda.

7. A method according to claim 1 **characterised in that** the 4-methoxybenzoic acid is precipitated from the reaction mixture of the oxidising ozonide separation by precipitation with a mineral acid in a 3-6 pH range and separated by filtration.

8. A method according to claim 1 **characterised in that** the reaction mixture is treated for eliminating side products after the oxidising separation of ozonide and before precipitation by mineral acid with activated carbon.

9. A method according to claim 1 **characterised in that** the 4-methoxybenzoic acid obtained from precipitation with a mineral acid by dissolution in diluted caustic soda and treatment with activated carbon and renewed precipitation is further purified with sulphuric acid.

## Revendications

1. Procédé de fabrication de acide 4-méthoxybenzoïque à partir de 1-méthoxy-4-(1-propényl)benzol (anéthole) **caractérisé en ce que** l'ozonide de l'anéthole est converti *in situ* en acide 4-méthoxybenzoïque par conversion oxydante avec du peroxyde d'hydrogène de base.

2. Procédé conformément à la revendication 1 **caractérisé en ce que** l'on obtient le produit de départ 1-méthoxy-4-(1-propényl)benzol (anéthole) grâce à des procédés d'extraction ou de distillation à partir de sources végétales.

3. Procédé conformément à la revendication 1 **caractérisé en ce que** la formation de l'ozonide à partir du 1-méthoxy-4-(1-propényl)benzol (anéthole) se produit à des températures comprises entre -30°C et +30°C dans un solvant parmi le groupe des acides C1-6 mono ou dicarboniques, ester, eau, acétone ou alcools C1-6 ou dans des mélanges à partir de ces solvants mentionnés plus haut.

4. Procédé conformément à la revendication 1 **caractérisé en ce que** la formation de l'ozonide à partir du 1-méthoxy-4-(1-propényl)benzol (anéthole) se produit à des températures comprises entre -10°C et +10°C dans l'éthanol.

5. Procédé conformément à la revendication 1 **caractérisé en ce que** pour réaliser la séparation oxydante de l'ozonide par rapport au 1-méthoxy-4-(1-propényl)benzol on mélange l'ozonide avec une solution de peroxyde d'hydrogène et l'on ajoute ensuite une solution diluée, à 30-105°C, d'un hydroxyde alcalin ou de terre alcaline.

6. Procédé conformément à la revendication 1 **caractérisé en ce que** pour réaliser la séparation oxydante de l'ozonide par rapport au 1-méthoxy-4-(1-propényl)benzol on mélange l'ozonide avec une solution de peroxyde d'hydrogène et l'on ajoute ensuite une solution de 1-25% de soude caustique à 60-80°C.

7. Procédé conformément à la revendication 1 **caractérisé en ce que** l'acide 4-méthoxybenzoïque est précipité du mélange de réaction de la séparation de l'ozonide oxydante par précipitation avec un acide minéral dans une fourchette d'acidité pH 3-6 avant séparation par filtration.

8. Procédé conformément à la revendication 1 **caractérisé en ce que** l'on traite le mélange de réaction pour éliminer les sous-produits après séparation oxydante de l'ozonide et avant précipitation par acide minéral avec du charbon actif.

9. Procédé conformément à la revendication 1 **caractérisé en ce que** l'on poursuit la purification de l'acide 4-méthoxybenzoïque obtenue à partir de la précipitation avec un acide minéral par dissolution dans de la soude caustique diluée et traitement avec du charbon actif et nouvelle précipitation avec de l'acide sulfurique.
